# FASCICULE DE BREVET EUROPEEN

(11) **EP 0 652 741 B1**
(45) Date de publication et mention de la délivrance du brevet: **09.10.1996**
(21) Numéro de dépôt: 93917840.6
(22) Date de dépôt: 29.07.1993
(51) Int. Cl.: A61K 7/08, A61K 7/13, A61K 7/135, A61K 7/50

(54) **COMPOSITIONS COSMETIQUES OU DERMATOLOGIQUES, EN PARTICULIER DES SHAMPOOINGS, DILUABLES A L'EAU SANS PERTE DE VISCOSITE APRES DILUTION**
KOSMETISCHE ODER DERMATOLOGISCHE MITTEL, INSBESONDERE SHAMPOOS, DIE OHNE VISKOSITÄTSVERLUSST NACH VERDÜNNUNG MIT WASSER VERDÜNNBAR SIND
COSMETIC OR DERMATOLOGICAL COMPOSITIONS, PARTICULARLY SHAMPOOS WHICH MAY BE DILUTED IN WATER WITHOUT LOSING VISCOSITY AFTER DILUTION

(30) Priorité: 03.08.1992 FR 9209617
(43) Date de publication de la demande: 17.05.1995
(73) Titulaire: L'OREAL, F-75008 Paris (FR)
(72) Inventeur: BEAUQUEY, Bernard, F-92111 Clichy (FR); DECOSTER, Sandrine, F-92400 Courbevoie (FR); VERITE, Claude, F-75014 Paris (FR)
(74) Mandataire: Casalonga, Axel
(86) Numéro de dépôt international: FR9300779
(87) Numéro de publication internationale: WO9403146

(56) Documents cités:
- EP-A- 0 019 970
- EP-A- 0 256 656
- FR-A- 1 574 317
- FR-A- 1 597 402
- FR-A- 2 622 205

## Description

La présente invention a pour objet des compositions cosmétiques ou dermatologiques, en particulier des shampooings pouvant être diluées à l'eau ou à l'eau oxygénée sans perte de viscosité.

On recherche dans le domaine de la cosmétique des compositions pouvant être utilisées telles quelles, notamment sous forme de shampooings, ou encore être utilisées comme supports de compositions cosmétiques tels que pour les traitements, notamment de colorations par oxydation des cheveux, qui présentent une viscosité permettant une application aisée et qui la conservent même après dilution.

La demanderesse a en effet constaté que si l'addition de sels ou d'amides permet d'augmenter rapidement et fortement la viscosité de compositions contenant des agents tensioactifs, cette viscosité est souvent trop élevée et pose des problèmes au niveau de l'utilisation, notamment au niveau de la dilution pour leur mise en oeuvre cosmétique. Ainsi lorsqu'on dilue ces compositions, on constate que leur viscosité chute presque linéairement pour atteindre une viscosité très faible, voire pratiquement nulle lorsque la dilution augmente jusqu'à 60 parties d'eau pour 100 parties de composition à base d'agents tensioactifs. De telles compositions présentent donc d'une part, des problèmes de mise en oeuvre, avant leur dilution, du fait de leur viscosité trop élevée et d'autre part des problèmes de mise en oeuvre après leur dilution, du fait de la chute rapide de la viscosité.

On recherche dans le domaine de la cosmétique, notamment dans le domaine des shampooings, des compositions concentrées nécessitant des volumes de conditionnement ou de stockage faibles, pouvant cependant être diluées très facilement sans apport d'une énergie mécanique particulière. Ces compositions doivent être homogènes après dilution et présenter une viscosité compatible avec une application cosmétique, c'est-à-dire une viscosité qui évite une fluidité trop importante entraînent un écoulement de la composition cosmétique lors de l'application.

La concentration et la réduction des volumes, tant au niveau du conditionnement que du stockage, présentent également des avantages économiques au niveau de la fabrication, puisqu'elles nécessitent des installations moins importantes. Ces compositions concentrées permettent de diminuer la concentration en conservateurs voire même de les supprimer.

La demanderesse a découvert, ce qui fait l'objet de l'invention, de nouvelles compositions présentant, en l'état concentré, une viscosité permettant leur dilution, et ayant, après dilution dans des proportions comprise entre 60 et 400 parties de diluant pour 100 parties de composition, une viscosité parfaitement compatible avec une application cosmétique.

L'invention a pour objet une composition cosmétique ou dermatologique utilisable telle quelle comme shampooing ou comme support pour des compositions de traitements cosmétiques notamment de colorations par oxydation des cheveux ou de traitement dermatologique, diluable sans perte de viscosité après dilution.

Un autre objet de l'invention est constitué par l'utilisation de ces compositions pour le lavage des cheveux et/ou de la peau ou pour la coloration des phanères.

D'autres objets de l'invention apparaîtront à la lecture de la description et des exemples suivants.

La composition conforme à l'invention est caractérisée par le fait qu'elle contient, dans un milieu aqueux cosmétiquement acceptable au moins :
- de 20 à 50 % en poids, par rapport au poids total de la composition d'un agent tensioactif constitué par un sel alcalin, d'ammonium ou d'amine d'un alkyléthersulfate oxyéthyléné ayant 1 à 7 moles d'oxyde d'éthylène et de préférence 25 à 35 % en poids de cet agent tensioactif.
- de 0,5 à 8 % en poids, par rapport au poids total de la composition, de monoéthanolamide d'acide gras en C₈-C₁₈ et de préférence 1 à 3 %
- du chlorure de sodium ou d'ammonium dans des proportions comprises entre 2 et 5 % en poids, le rapport entre le chlorure de sodium ou le chlorure d'ammonium et l'agent tensioactif étant compris entre 0,03 et 0,25, de préférence entre 0,07 et 0,18.
- la quantité en agent tensioactif, en monoéthanolamide et en chlorure de sodium et d'ammonium étant telle que la composition ait une viscosité, après dilution par 60 à 400 parties d'eau pour 100 g de composition, comprise entre 100 Pas et 0,5 Pas, mesurée à 25°C au Rhéomètre à contrainte imposée Carrimed CSL, pour une contrainte exprimée en N.m⁻² égale ou inférieure à 80.

Conformément à l'invention, le chlorure de sodium ou d'ammonium est utilisé de préférence dans des proportions comprises entre 2 et 3 % par rapport au poids total de la composition.

Les alkyléthersulfates oxyéthylénés ayant 1 à 7 moles d'oxyde éthylène utilisés de préférence sont les composés dans lesquels le groupe alkyle est linéaire ou ramifié et comporte de 9 à 15 atomes de carbone et de préférence des chaînes en C₁₂-C₁₄, et oxyéthylénés à 2 à 3 moles d'oxyde d'éthylène. Les produits préférés sont les produits vendus sous la dénomination TENSAGEX DLM 970 commercialisés par la société ICI ou sous la de,nomination TEXAPON N 70 par la société HENKEL.

La demanderesse a constaté que ces compositions pouvaient être facilement diluées par addition d'eau ou d'eau oxygénée sans nécessiter l'apport d'une énergie mécanique particulière; ces compositions, après dilution, restent homogènes.

Ces compositions présentent par ailleurs comme caractéristique dans leur forme de réalisation préférée d'avoir une vitesse d'écoulement d'au moins d'environ 20 s déterminée en Coupe Ford 10 à 25°C selon la norme AFNOR NF EN 535 avant ou après dilution.

Les agents tensioctifs utilisés conformément à l'invention peuvent être utilisés seuls ou en mélange avec un ou plusieurs co-tensioactifs. Ces co-tensioactifs peuvent être choisis parmi les tensioactifs anioniques, amphotères ou non-ioniques, différents des sels alcalins, d'ammonium, ou d'amines d'alkyléthersulfates oxyéthylénés ayant 1 à 7 moles d'oxyde d'éthylène définis ci-dessus.

Parmi ces agents tensioactifs on peut citer pour les agents tensioactifs anioniques les sels alcalins, d'ammonium, d'amines, d'aminoalcools ou de magnésium d'alkylsulfosuccinates dans lesquels le radical alkyle a de préférence 12 à 14 atomes de carbone, les paraffines sulfonates, les α-oléfines sulfonates, les acyl iséthionates, les alkyléthers carboxyliques polyoxyéthylénés, les acylaminoacides tels que acylpeptides, les acylsarcosinates, les N-acyltaurates.

Parmi les agents tensioactifs amphotères ou zwitterioniques, on peut citer les dérivés d'amines secondaires ou tertiaires aliphatiques, dans lesquels le radical aliphatique est une chaîne linéaire ou ramifiée comportant de 8 à 18 atomes de carbone et qui contient un groupe anionique hydrosolubilisant, carboxylate, sulfonate, sulfate, phosphate ou phosphonate les alkyl (C₈-C₂₀) bétaïnes, les sulfobétaïnes, les alkyl (C₈-C₂₀) amidoalkyl (C₁-C₆) bétaïnes, les alkyl (C₈-C₂₀) amidoalkyl (C₁-C₆) sulfobétaïnes ou les alkyl (C₈-C₂₀) amidoalkyl (C₁-C₆) hydroxyalkyl (C₁-C₆) sulfobétaïnes.

Parmi les dérivés d'amines, on peut citer les produits vendus sous la dénomination "MIRANOL", tels que décrits dans brevets US-A-2 528 378 et 2 781 354 ou classés dans le dictionnaire CTFA, 3ème édition, 1982, sous la dénomination d'Amphocarboxyglycinates ou d'Amphocarboxypropionates.

Parmi les agents tensioactifs non ioniques, on utilise de préférence les alkyl (C₈-C₂₄) polyglucosides tels que les produits vendus sous la dénomination APG par la société HENKEL comme par exemple les produits APG 300, APG 350, APG 500, APG 550, APG 625, APG base 10-12; les produits vendus par la société SEPPIC sous les dénominations TRITON CG 110 (ou ORAMIX CG 110) et TRITON CG 312 (ou ORAMIX NS 10), ou ceux vendus par la société BASF sous la dénomination LUTENSOL GD 70.

On peut également utiliser des alcools à chaîne grasse comportant 8 à 18 atomes de carbone, polyoxyéthylénés avec un nombre d'oxyde d'éthylène supérieur à 10 ou des polyhydroxypropyléthers tels que ceux décrits ou préparés selon les procédés des brevets FR -1 477 048, 2 328 763, 2 091 516, 2 169 787, 2 574 786.

Les agents tensioactifs utilisés conjointement avec les alkyléthers sulfates oxyéthylénés, présents dans les compositions conformes à l'invention, peuvent représenter jusqu'à 50 %, en poids, de la quantité totale d'agents tensioactifs présents dans la composition. La quantité totale d'agents tensioactifs est comprise entres 20 et 50% en poids par rapport au poids total de la composition.

Selon une forme préférée de réalisation de l'invention, on utilise conjointement avec les sels alcalins, d'ammonium ou d'amines d'alkyléthersulfates oxyéthylénés ayant 1 à 7 moles d'oxyde d'éthylène, des agents tensioactifs amphotères ou zwitterioniques et notamment les dérivés de bétaïne mentionnés ci-dessus.

On utilise de façon préférée dans les compositions conformes à l'invention les monoéthanolamides d'acides de coprah naturel ou synthétique.

La composition conforme à l'invention contient de préférence le chlorure de sodium.

Les compositions conformes à l'invention peuvent être utilisées telles quelles ou avec des additifs habituellement utilisés en cosmétique ou en dermatologie comme compositions lavantes pour les cheveux et/ou la peau. Elles peuvent contenir plus particulièrement des additifs qui n'altèrent pas les propriétés à la dilution définie ci-dessus choisis parmi les agents tensioactifs cationiques, les polymères anioniques, non-ioniques cationiques ou amphotères, les protéines animales ou végétales quaternisées ou non, des huiles hydrocarbonées telle que des huiles de synthèse comme les isoparaffines ou les huiles minérales végétales ou animales, des huiles, cires, résines ou gommes de silicone, des agents acidifiants ou alcalinisants ou des tampons, des agents conservateurs, des agents adoucissants, des actifs cosmétiques ou dermatologiques, des filtres solaires, des agents nacrants, des agents opacifiants, la di ou polyéthanolamide de coprah, l'amide d'acides de colza oxyéthyléné à 3 moles d'oxyde d'éthylène des alcools polyoxyéthylénés à 2 à 3 moles d'oxyde d'éthylène, des parfums et de tous autres adjuvants habituellement utilisés dans des compositions cosmétiques ou dermatologiques.

La demanderesse a constaté que ces compositions, une fois diluées à l'eau dans les proportions indiquées conservaient non seulement une bonne viscosité rendant leur utilisation particulièrement facile pour le lavage des cheveux et de la peau mais conservaient également un excellent pouvoir moussant.

Selon une autre forme de réalisation de l'invention, les compositions telles que définies ci-dessus peuvent être utilisées comme supports de colorants d'oxydation des cheveux. Les colorants qui peuvent être introduits dans ces compositions sont connus en eux-mêmes, et sont des précurseurs de colorants d'oxydation formant des colorants à la suite d'un processus de condensation oxydative de ces précurseurs en présence éventuellement de coupleurs ou modificateurs.

On peut également utiliser des colorants tels que des colorants indoliques générant des pigments de type mélanique sous l'action d'un agent oxydant, par exemple de l'eau oxygénée.

Les précurseurs de colorants d'oxydation utilisables sont entre autre décrits dans l'ouvrage ZVIAK - Science des Traitements Capillaires - Masson - 1988 - pages 235 à 287. Il s'agit plus particulièrement de diamines ou d'aminophénols comportant des groupements fonctionnels amino et hydroxy en position para ou ortho. Les coupleurs ou modificateurs sont plus particulièrement des métadiamines, des méta-aminophénols ou des métadiphénols.

Les colorants indoliques générant des pigments du type mélanique et leurs agents oxydants sont par exemple décrits dans les demandes de brevets et brevets FR-A- 2593 061, 2 593 062, 2 659 552, 2 606 636, 2 636 236, 2 624 730, 2 654 335, et les demandes de brevets européens EP-A 424 261, EP-A 446 132. Les composés indoliques préférés sont le 5,6-dihydroxyindole et ses dérivés et les 6 et 7-monohydroxyindoles.

Ces compositions peuvent également contenir, en plus des précurseurs de colorants d'oxydation ou des colorants indoliques, des colorants directs qui permettent d'enrichir les nuances tels que les colorants azoïques, anthraquinoniques les dérivés nitrés de la série benzoniques, les indamines, indoanilines et indophénols. Ces colorants sont également décrits dans l'ouvrage Science des Traitements Capillaires mentionné ci-dessus.

Ces compositions tinctoriales sont diluées avec une solution aqueuse d'eau oxygénée en vue de développer la coloration avant leur application sur les cheveux. On mélange généralement entre 60 et 300 parties d'une solution aqueuse de peroxyde hydrogène à 100 parties de la composition tinctoriale contenant les précurseurs de colorants. La demanderesse a constaté, également dans ce cas, que la viscosité après dilution répondait aux caractéristiques définies ci-dessus, et permettait une teinture des cheveux sans écoulement excessif de la composition tinctoriale sur les parties ne devant pas être teintes.

Les exemples suivants sont destinés à illustrer l'invention sans présenter un caractère limitatif.

### EXEMPLE 1

Shampooing concentré pour les cheveux et le corps.

| | |
|---|---|
| Alkyl (C₉/C₁₁/C₁₃/C₁₅ : 3/7/63/27 à 50 % linéaires) éther sulfate de sodium 3. moles d'oxyde d'éthylène en solution aqueuse à 70 % MA sous la dénomination TENSAGEX DLM 970 par la société ICI | 20 g MA |
| Alkyl (C₉/C₁₀/C₁₁/- 20/40/40) - polyglucoside (1,4) vendu sous la dénomination APG 300 à 50 % de MA par la société HENKEL | 12,5 g MA |
| Polymère d'hydroxyéthylcellulose et d'épichlorhydrine quaternisée avec la triméthylamine, vendu sous la dénomination JR400 par la société UNION CARBIDE | 0,2 g |
| Monoéthanolamide d'acide de coprah vendu sous la dénomination COMPERLAN 100 par la société HENKEL | 6 g |
| Chlorure de sodium | 2,53 g * |
| Parfum, colorants, séquestrants, conservateurs | qs |
| HCI qs : pH : 7 | |
| Eau qsp | 100 g |

| | |
|---|---|
| * les 2,53 g de chlorure de sodium correspondent au chlorure de sodium contenu dans le TENSAGEX DLM 970 (0,03 g) et au chlorure de sodium ajouté (2,5 g) | |

### EXEMPLE 2

Shampooing concentré pour les cheveux et le corps.

| | |
|---|---|
| Alkyl (C₉/C₁₁/C₁₃/C₁₅ : 3/7/63/27 à 50 % linéaires) éther sulfate de sodium 3. moles d'oxyde d'éthylène vendu en solution aqueuse à 70 % MA sous la dénomination TENSAGEX DLM 970 par la société ICI | 23,1 g MA |
| Alkyl (C₉/C₁₀/C₁₁/- 20/40/40) - polyglucoside (1,4) vendu sous la dénomination APG 300 à 50 % de MA par la société HENKEL | 6,3 g MA |
| Polymère d'hydroxyéthylcellulose et d'épichlorhydrine quaternisée avec la triméthylamine, vendu sous la dénomination JR400 par la société UNION CARBIDE | 0,2 g |
| Monoéthanolamide d'acide de coprah vendu sous la dénomination COMPERLAN 100 par la société HENKEL | 6 g |
| Chlorure de sodium | 2,53 g * |
| Parfum, colorants, séquestrants, conservateurs | qs |
| HCI qs : pH : 7 | |
| Eau qsp | 100 g |

| | |
|---|---|
| * les 2,53 g de chlorure de sodium correspondent au chlorure de sodium contenu dans le TENSAGEX DLM 970 (0,03 g) et au chlorure de sodium ajouté (2,5 g) | |

### EXEMPLE 3

Shampooing concentré pour les cheveux.

| | |
|---|---|
| Alkyl (C₉/C₁₁/C₁₃/C₁₅ : 3/7/63/27 à 50 % linéaires) éther sulfate de sodium 3. moles d'oxyde d'éthylène vendu en solution aqueuse à 70 % MA sous la dénomination TENSAGEX DLM 970 par la société ICI | 24,5 g MA |
| Hydroxyde d'alkyl (C₁₂-C₁₈) diméthyl carboxyméthyl ammonium vendu à 30 % de matière active (MA) sous la dénomination DEHYTON A 30 par la société HENKEL | 4,5 g MA |
| Monoéthanolamide d'acide de coprah vendu sous la dénomination COMPERLAN 100 par la société HENKEL | 1 g |
| Chlorure de sodium | 4,43 g* |
| Parfum, colorants, séquestrants, conservateurs | qs |
| pH spontané : 6,7 | |
| Eau qsp | 100 g |

| | |
|---|---|
| * les 4,43 g de chlorure de sodium correspondent au chlorure de sodium contenu dans le TENSAGEX DLM 970 (0,03 g) et dans le DEHYTON A 30 (0,9 g) et au chlorure de sodium ajouté (3,5 g) | |

### EXEMPLE 4

Shampooing concentré pour les cheveux.

| | |
|---|---|
| Alkyl (C₉/C₁₁/C₁₃/C₁₅ : 3/7/63/27 à 50 % linéaires) éther sulfate de sodium 3. moles d'oxyde d'éthylène vendu en solution aqueuse à 70 % MA sous la dénomination TENSAGEX DLM 970 par la société ICI | 22,4 g MA |
| Parrafine (C₁₂-C₁₆) sulfonate de sodium vendu en solution aqueuse à 30 % sous la dénomination HOSTAPUR SAS 30 par la société HOECHST | 7,5 g MA |
| Copolymère de polyvinylpyrrolidone quaternisé ayant un PM de 1 000 000 vendu à 20 % MA par la société GAF sous la dénomination GAFQUAT 755 | 0,4 G MA |
| Monoéthanolamide d'acide de coprah vendu sous la dénomination COMPERLAN 100 par la société HENKEL | 3 g |
| Chlorure de sodium | 3,03 g* |
| Parfum, colorants, séquestrants conservateurs | qs |
| pH spontané : 7,3 | |
| Eau qsp | 100 g |

| | |
|---|---|
| * les 3,03 g de chlorure de sodium correspondent au chlorure de sodium contenu dans le TENSAGEX DLM 970 (0,03 g) et au chlorure de sodium ajouté (3 g) | |

### EXEMPLE 5

On a préparé la composition suivante :

| | |
|---|---|
| Lauryléther sulfate de sodium (C₁₂/C₁₄) 70/30 à 2,2 mole d'oxyde d'éthylène en solution aqueuse à 70 % vendu sous la dénomination TEXAPON N 70 par la société HENKEL | 27,3 g (MA) |
| Monoéthanolamine d'acide de coprah | 4,0 g |
| Chlorure de sodium | 3,54* g |
| Eau qsp | 100 g |

| | |
|---|---|
| * les 3,54 g de chlorure de sodium correspondent au chlorure de sodium contenu dans le TEXAPON N70 (0,04 g) et au chlorure de sodium ajouté (3,5 g). | |

On constate que la composition avant dilution a une viscosité de 2,2 PAS; une viscosité de 62,6 PAS après dilution avec 67 parties d'eau pour 100 parties de composition, et une viscosité de 7,3 PAS après une dilution de 100 parties d'eau pour 100 parties de composition; ces mesures étant effectuées à 25°C au Rhéomètre à contraintre imposée Carrimed CSL pour une contrainte exprimée en N.m⁻² égale à 40.

### EXEMPLE 6

On prépare la composition suivante :

| | |
|---|---|
| Alkyléther sulfate de sodium (C₉/C₁₁/C₁₃/C₁₅ : 3/7/63/27 à 50 % linéaires) à 3 moles d'oxyde éthylène en solution aqueuse à 70 % vendu sous la dénomination TENSAGEX DLM 970 par la société ICI | 24,5 g MA |
| Monoéthanolamide d'acide de coprah | 3,0 g MA |
| Mélange cocoyl amidopropylebetaïne/monolaurate de glycérol 25/5 en solution aqueuse à 30 % vendu sous la dénomination TEGOBETAINE HS par la société GOLDSCHMIDT | 4,5 g MA |
| Chlorure de sodium | 2,8 g * |
| Eau qsp | 100 g |

| | |
|---|---|
| * Les 2,8 g de chlorure de sodium également au chlorure de sodium contenu dans le TENSAGEX DLM 970 (0,05 g) et dans la TEGOBETAINE HS (0,75 g) et au chlorure de sodium ajouté. | |

Cette composition a une viscosité de 2,1 PAS avant dilution. Pour une dilution à 67 parties d'eau pour 100 parties de composition, on constate une viscosité 86,5 PAS et pour une dilution de 100 parties d'eau pour 100 parties de composition, on a une viscosité de 10,7 PAS. Ces viscosités sont mesurées à 25°C au Rhéomètre à contrainte imposée Carrimed CSL pour une contrainte exprimée en N.m⁻² égale à 50.

### EXEMPLE 7

On prépare la composition suivante :

| | |
|---|---|
| Alkyléther sulfate de sodium (C9/C11/C13/C15) : 3/7/63/27 à 50 % linéaires) oxyéthyléné à 3 moles d'oxyde d'éthylène en solution aqueuse à 70 % vendu sous la dénomination TENSAGEX DLM 970 par la société ICI | 28 g MA |
| Monoéthanolamide d'acide de coprah | 3 g MA |
| Chlorure d'ammonium | 3 g |
| Chlorure de sodium* | 0,04 g |
| Acide chlorhydrique qs : pH : 7 | |
| - Eau qsp | 100 g |

| | |
|---|---|
| * correspond au chlorure de sodium contenu dans le TENSAGEX DLM 970. | |

La composition est utilisée comme shampooing concentré pour les cheveux.

### EXEMPLE 8

On prépare la composition suivante :

| | |
|---|---|
| Alkyl (C9/C11/C13/C15 : 3/7/63/27 à 50 % linéaires) éther sulfate de sodium oxyéthyléné à 3 moles d'oxyde d'éthylène en solution aqueuse à 70 % vendu sous la dénomination TENSAGEX DLM 970 par la société ICI | 35 g MA |
| Monoéthanolamide d'acide de coprah | 1 g MA |
| Chlorure de sodium* | 5 g |
| ICI qs : pH : 7 | |
| Eau qs | 100 g |

| | |
|---|---|
| * Les 5,00 g de chlorure de sodium correspondent au chlorure de sodium contenu dans le TENSAGEX DLM 970 (0,05 g) et au chlorure de sodium ajouté (4,95 g). | |

Cette composition est utilisée comme shampooing concentré pour les cheveux.

### EXEMPLE 9

On prépare la composition suivante :

| | |
|---|---|
| Alkyl (C9/C11/C13/C15 : 3/7/63/27 a 50 % linéaires) éther sulfate de sodium oxyéthyléné à 3 moles d'oxyde d'éthylène vendu en solution aqueuse à 70 % MA sous la dénomination TENSAGEX DLM 970 par la société ICI | 28 g MA |
| Monoéthanolamide d'acide de coprah vendu sous la dénomination COMPERLAN 100 par la société HENKEL | 5 g |
| Parfum, colorants, séquestrants, conservateurs | qs |
| Chlorure de sodium * | 2,04 g |
| HCl qs : pH : 7 | |
| Eau qs | 100 g |

| | |
|---|---|
| * Les 2,04 g de chlorure de sodium correspondent au chlorure de sodium contenu dans le TENSAGEX DLM (0,04 g) et au chlorure de sodium ajouté (2,0 g). | |

Cette composition est utilisée comme shampooing concentré pour les cheveux.

### EXEMPLE 10

| | |
|---|---|
| Alkyl (C9/C11/C13/C15 : 3/7/63/27 à 50 % linéaires) éther sulfate de sodium oxyéthyléné à 3 moles d'oxyde d'éthylène vendu en solution aqueuse à 70 % MA sous la dénomination TENSAGEX DLM 970 par la société ICI | 28 g MA |
| Monoéthanolamide d'acide de coprah vendu sous la dénomination COMPERLAN 100 par la société HENKEL | 5 g |
| Chlorure de sodium* | 3, 04 g |
| Paraphénylène diamine | 0,5 g |
| Métadihydroxybenzène | 0,5 g |
| Ammoniaque à 20% en solution aqueuse | 10 g |
| Métabisulfite de sodium en solution aqueuse à 35 % MA | 0,46 g MA |
| Parfum, colorants, séquestrants, conservateurs | |
| pH spontané : 10 | |
| Eau qs | 100 g |

| | |
|---|---|
| * Les 3,04 g de chlorure de sodium correspondent au chlorure de sodium contenu dans le TENSAGEX DLM 970 (0,04 g) et au chlorure de sodium ajouté (3 g). | |

Cette composition est utilisée pour la coloration d'oxydation des cheveux. Les 100 parties de ce support sont diluées au moment de l'emploi avec 60 parties d'eau oxygénée (20 volumes).

## Revendications

1. Composition cosmétique ou dermatologique caractérisée par le fait qu'elle contient dans un milieu aqueux cosmétiquement acceptable au moins :
- de 20 à 50% en poids d'un agent tensioactif constitué par un sel alcalin, d'ammonium ou d'amine d'un alkyléthersulfate oxyéthyléné ayant 1 à 7 moles d'oxyde d'éthylène,
- de 0,5 à 8 % en poids de monoéthanolamide d'acide gras en C₈-C₁₈
- du chlorure de sodium ou d'ammonium dans des proportions pondérales comprises entre 2 et 5 %, le rapport entre le chlorure de sodium ou le chlorure d'ammonium et l'agent tensioactif étant compris entre 0,03 et 0,25,
la quantité en agents tensioactifs, en monoéthanolamide et en chlorure de sodium ou d'ammonium étant telle que la composition a une viscosité après dilution par 60 et 400 parties d'eau pour 100 g de composition, comprise entre 100 PAS et 0,5 PAS mesurée à 25°C au Rhéomètre à contrainte imposée Carrimed CSL pour une contrainte exprimée en N.m⁻² égale ou inférieure à 80.

2. Composition selon la revendication 1, caractérisée par le fait que l'agent tensioactif est présent dans des proportions comprises entre 25 et 35 % en poids par rapport au poids total de la composition.

3. Composition selon la revendication 1 ou 2, caractérisée par le fait que le monoéthanolamide d'acide gras en C₈-C₁₈ est présent dans des proportions comprises entre 1 et 3 % en poids par rapport au poids total de la composition.

4. Composition selon l'une quelconque des revendications 1 à 3, caractérisée par le fait que le chlorure de sodium ou d'ammonium est présent dans des proportions comprises entre 2 et 3 % en poids par rapport au poids total de la composition.

5. Composition selon l'une quelconque des revendications 1 à 4, caractérisée par le fait que la composition a une vitesse d'écoulement supérieure à 20 s avant ou après dilution.

6. Composition selon l'une quelconque des revendications 1 à 5, caractérisée par le fait qu'elle contient également des agents tensioactifs différents des sels alcalins, d'ammonium ou d'amines d'alkyléthersulfates oxyéthylénés ayant de 1 à 7 moles d'oxyde d'éthylène choisis parmi les agents tensioactifs anioniques amphotères ou non-ioniques.

7. Composition selon la revendication 6, caractérisée par le fait qu'e les agents tensioactifs anioniques sont choisis parmi les sels alcalins, d'ammonium, d'amines, d'aminoalcools ou de magnésium d'alkylsulfosuccinates, de paraffine sulfonates, d'α-oléfines sulfonates, d'acyl iséthionates, d'alkyléthercarboxyliques polyoxyéthylénés, d'acylaminoacides.

8. Composition selon la revendication 6, caractérisée par le fait que les agents amphotères ou zwitterioniques sont choisis parmi les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant, carboxylate, sulfonate, sulfate, phosphate ou phosphonate, les alkyl (C₈-C₂₀) bétaïnes, les sulfobétaïnes, les alkyl (C₈-C₂₀) amidoalkyl (C₁-C6) bétaïnes, les alkyl (C₈-C₂₀) amidoalkyl (C₁-C₆) sulfobétaïnes ou les alkyl C₈-C₂₀ amidoalkyl (C₁-C₆) hydroxyalkyl (C₁-C₆) sulfobétaïnes.

9. Composition selon la revendication 6, caractérisée par le fait que les agents tensioactifs non ioniques sont choisis parmi les alkyl (C₈-C₂₄) polyglucosides, les alcools à chaîne grasse comportant 8 à 18 atomes de carbone polyoxyéthylénés avec un nombre d'oxyde d'éthylène supérieur à 10 ou des polyhydroxypropyléthers.

10. Utilisation de la composition telle que définie dans l'une quelconque des revendications 1 à 9 pour le lavage des cheveux et/ou de la peau après dilution de la composition avec 60 à 400 parties d'eau pour 100 g de composition.

11. Composition selon l'une quelconque des revendications 1 à 9, caractérisée par le fait qu'elle contient des précurseurs de colorants d'oxydation et/ou des colorants indoliques générant des pigments mélaniques.

12. Utilisation de la composition selon la revendication 11 pour la coloration par oxydation des cheveux, caractérisée par le fait que l'on ajoute à la composition entre 60 et 300 parties d'eau oxygénée pour 100 parties de composition tinctoriale et qu'on procède ensuite à la teinture des cheveux.

## Claims

1. Cosmetic or dermatological composition, characterized in that it contains, in a cosmetically acceptable agueous medium, at least:
- from 20 to 50% by weight of a surface-active agent consisting of an alkali metal, ammonium or amine salt of an oxyethylenated alkyl ether sulfate having 1 to 7 mol of ethylene oxide,
- from 0.5 to 8% by weight of C₈-C₁₈ fatty acid monoethanolamide,
- sodium or ammonium chloride in proportions by weight of between 2 and 5%, the ratio of the sodium chloride or the ammonium chloride to the surface-active agent being between 0.03 and 0.25,
- the amount of surface-active agents, of monoethanolamide and of sodium or ammonium chloride being such that the composition has a viscosity, after dilution with 60 and 400 parts of water per 100 g of composition, of between 100 Pa·s and 0.5 Pa·s, measured at 25°C with a controlled stress Carrimed CSL Rheometer, for a stress, expressed in N.m⁻², equal to or less than 80.

2. Composition according to Claim 1, characterized in that the surface-active agent is present in proportions of between 25 and 35% by weight with respect to the total weight of the composition.

3. Composition according to Claim 1 or 2, characterized in that the C₈-C₁₈ fatty acid monoethanolamide is present in proportions of between 1 and 3% by weight with respect to the total weight of the composition.

4. Composition according to any one of Claims 1 to 3, characterized in that the sodium or ammonium chloride is present in proportions of between 2 and 3% by weight with respect to the total weight of the composition.

5. Composition according to any one of Claims 1 to 4, characterized in that the composition has a flow rate which is greater than 20 s, before or after dilution.

6. Composition according to any one of Claims 1 to 5, characterized in that it also contains surface-active agents other than the alkali metal, ammonium or amine salts of oxyethylenated alkyl ether sulfates having from 1 to 7 mol of ethylene oxide chosen from anionic, amphoteric or nonionic surface-active agents.

7. Composition according to Claim 6, characterized in that the anionic surface-active agents are chosen from alkali metal, ammonium, amine, aminoalcohol or magnesium salts of alkyl sulfosuccinates, paraffin sulfonates, α-olefin sulfonates, acyl isethionates, polyoxyethylenated alkyl ether carboxylics or acylamino acids.

8. Composition according to Claim 6, characterized in that the amphoteric or zwitterionic agents are chosen from the derivatives of secondary or tertiary aliphatic amines in which the aliphatic radical is a linear or branched chain containing 8 to 18 carbon atoms and which contains at least one carboxylate, sulfonate, sulfate, phosphate or phosphonate, water-solubilizing anionic group, alkyl (C₈-C₂₀) betaines, sulfobetaines, alkyl (C₈-C₂₀) amidoalkyl (C₁-C6) [sic] betaines, alkyl (C₈-C₂₀) amidoalkyl (C₁-C₆) sulfobetaines or alkyl C₈-C₂₀ amidoalkyl (C₁-C₆) hydroxyalkyl (C₁-C₆) sulfobetaines.

9. Composition according to Claim 6, characterized in that the nonionic surface-active agents are chosen from alkyl (C₈-C₂₄) polyglucosides, alcohols with a fatty chain containing 8 to 18 carbon atoms which are polyoxyethylenated with an ethylene oxide number greater than 10 or poly(hydroxypropyl ether)s.

10. Use of the composition as defined in any one of Claims 1 to 9 for washing the hair and/or the skin after dilution of the composition with 60 to 400 parts of water per 100 g of composition.

11. Composition according to any one of Claims 1 to 9, characterized in that it contains oxidation dye precursors and/or indole dyes which generate melanin pigments.

12. Use of the composition according to Claim 11 for oxidation hair dyeing, characterized in that between 60 and 300 parts of hydrogen peroxide per 100 parts of dyeing composition are added to the composition and in that the hair is then dyed.

## Patentansprüche

1. Kosmetische oder dermatologische Zusammensetzung,
dadurch **gekennzeichnet**, daß
sie in einem kosmetisch geeigneten wässrigen Milieu mindestens enthält:
- 20 bis 50 Gew.% eines oberflächenaktiven Mittels aus einem Alkali-, Ammonium- oder Aminsalz eines mit 1 bis 7 Mol Ethylenoxid oxethylierten Alkylethersulfats,
- 0,5 bis 8 Gew. % Monoethanolamid einer C₈₋₁₈-Fettsäure,
- Natrium- oder Ammoniumchlorid in Gewichtsanteilen von 2 bis 5%, wobei das Verhältnis vom Natrium- oder Ammoniumchlorid zum oberflächenaktiven Mittel 0,03 bis 0,25 beträgt,
und wobei die Menge an oberflächenaktivem Mittel, an Monoethanolamid und an Natrium- oder Ammoniumchlorid so bemessen ist, daß die Zusammensetzung nach Verdünnen mit 60 bis 100 Teilen Wasser pro 100 g Zusammensetzung, eine Viskosität von 100 bis 0,5 Pa x s aufweist, gemessen bei 25°C mit dem Spannungs-Rheometer Carrimed CSL bei einer in N x m⁻² angegebenen Spannung von 80 oder weniger.

2. Zusammensetzung gemäß Anspruch 1,
dadurch **gekennzeichnet**, daß
das oberflächenaktive Mittel in Mengenanteilen von 25 bis 35 Gew.% vorhanden ist, bezogen auf das Gesamtgewicht der Zusammensetzung.

3. Zusammensetzung gemäß Anspruch 1 oder 2,
dadurch **gekennzeichnet**, daß
das Monoethanolamid einer C₈₋₁₈-Fettsäure in Mengenanteilen von 1 bis 3 Gew.% vorhanden ist, bezogen auf das Gesamtgewicht der Zusammensetzung.

4. Zusammensetzung gemäß einem der Ansprüche 1 bis 3,
dadurch **gekennzeichnet**, daß
das Natrium- oder Ammoniumchlorid in Mengenanteilen von 2 bis 3 Gew.% vorhanden sind, bezogen auf das Gesamtgewicht der Zusammensetzung.

5. Zusammensetzung gemäß einem der Ansprüche 1 bis 4,
dadurch **gekennzeichnet**, daß
die Zusammensetzung eine Ausgießgeschwindigkeit von mehr als 20 s vor oder nach Verdünnen aufweist.

6. Zusamemnsetzung gemäß einem der Ansprüche 1 bis 5,
dadurch **gekennzeichnet**, daß
sie auch oberflächenaktive Mittel enthält, die sich von den Alkali-, Ammonium- oder Aminsalzen der mit 1 bis 7 Mol Ethylenoxid oxethylierten Alkylethersulfate unterscheiden und aus anionischen, amphoteren oder nicht-ionischen oberflächenaktiven Mitteln ausgewählt sind.

7. Zusammensetzung gemäß Anspruch 6,
dadurch **gekennzeichnet**, daß
die anionischen oberflächenaktiven Mittel aus Alkali-, Ammonium-, Amin-, Aminoalkohol- oder Magnesiumsalzen von Alkylsulfosuccinaten, aus Paraffinsulfonaten, α-Olefinsulfonaten, Acylisethionaten, polyoxethylierten Alkylethercarboxylsäuren und aus Acylaminosäuren ausgewählt sind.

8. Zusammensetzung gemäß Anspruch 6,
dadurch **gekennzeichnet**, daß
die amphoteren oder zwitterionischen Mittel aus Derivaten sekundärer oder tertiärer aliphatischer Amine, in denen der aliphatische Rest eine lineare oder verzweigte Kette mit 8 bis 18 Kohlenstoffatomen ist, welcher mindestens eine anionische wasserlösliche Carboxylat-, Sulfonat-, Sulfat-, Phosphat- oder Phosphonat-Gruppe enthält, aus C₈₋₂₀-Alkylbetainen, Sulfobetainen, C₈₋₂₀-Alkylamido-C₁₋₆-alkylbetainen, C₈₋₂₀-Alkylamido-C₁₋₆-alkylsulfobetainen oder aus C₈₋₂₀-Alkylamido-C₁₋₆-alkylhydroxy-C₁₋₆-alkylsulfobetainen ausgewählt sind.

9. Zusamensetzung gemäß Anspruch 6,
dadurch **gekennzeichnet**, daß
die nicht-ionichen oberflächenaktiven Mittel aus C₈₋₂₄-Alkylpolyglucosiden, aus mit einer Anzahl von mehr als 10 Ethylenoxiden polyoxethylierten Alkoholen mit einer Fettkette von 8 bis 18 Kohlenstoffatomen oder aus Polyhydroxypropylethern ausgewählt sind.

10. Verwendung der in einem der Ansprüche 1 bis 9 definierten Zusammensetzung zum Waschen der Haare und/oder der Haut nach Verdünnung der Zusammensetzung mit 60 bis 400 Teilen Wasser pro 100 g Zusammensetzung.

11. Zusammensetzung gemäß jedem der Ansprüche 1 bis 9,
dadurch **gekennzeichnet**, daß
sie Oxidationsfarbstoff-Vorstufenverbindungen und/oder Melanin-Pigmente erzeugende Indol-Farbstoffe enthält.

12. Verwendung der Zusammensetzung gemäß Anspruch 11 zur Oxidationsfärbung der Haare,
dadurch **gekennzeichnet**, daß
man der Zusammensetzung 6 bis 300 Teile sauerstoffhaltiges Wasser pro 100 Teile Färbezusammensetzung zufügt und dann die Färbung der Haare durchführt.
